# EUROPEAN PATENT SPECIFICATION

(11) **EP 3 510 649 B1**
(45) Date of publication and mention of the grant of the patent: **21.10.2020**
(21) Application number: 17777970.9
(22) Date of filing: 07.09.2017
(51) Int. Cl.: H01M 2/10, A24F 47/00, H01M 4/38, H01M 4/485, H01M 4/505, H01M 4/525, H01M 4/587, H01M 10/052

(54) **POWER SOURCE FOR AN AEROSOL DELIVERY DEVICE**
NETZTEIL FÜR EINE AEROSOLABGABEVORRICHTUNG
SOURCE D'ALIMENTATION POUR UN DISPOSITIF DE DISTRIBUTION D'AÉROSOL

(30) Priority: 09.09.2016 US 201615261329
(43) Date of publication of application: 17.07.2019
(73) Proprietor: RAI Strategic Holdings, Inc., Winston-Salem, NC 27101 (US)
(72) Inventor: PHILLIPS, Percy, Pfafftown, North Carolina 27040 (US); SEBASTIAN, Andries Don, Winston-Salem, NC 27103 (US); DAVIS, Michael F., Clemmons, North Carolina 27012 (US); AMPOLINI, Frederic, Winston-Salem, North Carolina 27106 (US); SEARS, Stephen B., Siler City, North Carolina 27344 (US)
(74) Representative: Hoeger, Stellrecht & Partner Patentanwälte mbB
(86) International application number: PCT/IB2017/055401
(87) International publication number: WO 2018/047097

(56) References cited:
- WO-A1-2014/102092
- WO-A1-2016/066632
- WO-A1-2017/033007
- WO-A2-2009/066916
- US-A1- 2014 270 727

## Description

### TECHNOLOGICAL FIELD

The present disclosure relates to aerosol delivery devices such as smoking articles, and more particularly to aerosol delivery devices that may utilize electrically generated heat for the production of aerosol (e.g., smoking articles commonly referred to as electronic cigarettes). The smoking articles may be configured to heat an aerosol precursor, which may incorporate materials that may be made or derived from, or otherwise incorporate tobacco, the precursor being capable of forming an inhalable substance for human consumption.

### BACKGROUND

Many smoking devices have been proposed through the years as improvements upon, or alternatives to, smoking products that require combusting tobacco for use. Many of those devices purportedly have been designed to provide the sensations associated with cigarette, cigar or pipe smoking, but without delivering considerable quantities of incomplete combustion and pyrolysis products that result from the burning of tobacco. To this end, there have been proposed numerous smoking products, flavor generators and medicinal inhalers that utilize electrical energy to vaporize or heat a volatile material, or attempt to provide the sensations of cigarette, cigar or pipe smoking without burning tobacco to a significant degree. See, for example, the various alternative smoking articles, aerosol delivery devices and heat generating sources set forth in the background art described in U.S. Pat. Nos. 7,726,320 to Robinson et al. and 8,881,737 to Collett et al. See also, for example, the various types of smoking articles, aerosol delivery devices and electrically-powered heat generating sources referenced by brand name and commercial source in U.S. Pat. Pub. No. 2015/0216232 to Bless et al. Additionally, various types of electrically powered aerosol and vapor delivery devices also have been proposed in U.S. Pat. Pub. Nos. 2014/0096781 to Sears et al. and 2014/0283859 to Minskoff et al., as well as U.S. Pat. App. Ser. Nos. 14/282,768 to Sears et al., filed May 20, 2014; 14/286,552 to Brinkley et al., filed May 23, 2014; 14/327,776 to Ampolini et al., filed July 10, 2014; and 14/465,167 to Worm et al.

It would be desirable to provide aerosol delivery devices that utilize lithium-ion batteries (LiB) as a power source.

From US 2014/270727 A1, a method for controlling heating of an aerosol precursor arrangement of an electronic smoking article is known. An average power is directed from a power source to a heating device arranged to heat the aerosol precursor arrangement and a heating time period commensurately initiated. The average power corresponds to a selected power set point associated with the power source. An actual power directed to the heating device is determined as a product of a voltage at, and a current through, the heating device. The actual power is compared to the average power, and the average power is adjusted to direct the actual power toward the selected power set point. The actual power is periodically determined and compared to the average power, and the average power adjusted toward the selected power set point, until expiration of the heating time period.

From WO 2014/102092 A1, a heating assembly for heating an aerosol-forming substrate is known. The heating assembly comprises a heater comprising an electrically resistive heating element and a heater substrate and a heater mount coupled to the heater. The heating element comprises a first portion and a second portion configured such that, when an electrical current is passed through the heating element, the first portion is heated to a higher temperature than the second portion as a result of the electrical current. The heater mount surrounds the second portion of the heating element.

From WO 2016/066632 A1, a method of charging a second battery from a first battery is known. The method comprises comparing an output voltage of the first battery with a threshold voltage, when the output voltage from the first battery is equal to or greater than the threshold voltage, charging the second battery using a first current, and when the output voltage from the first battery is less than the threshold voltage, reducing the first current until the output voltage of the first battery is equal to or greater than a second threshold voltage.

From WO 2009/066916 A2, a separator is known, comprising a porous substrate having a plurality of pores and a porous coating layer formed on at least one surface of the porous substrate and made of a mixture of a plurality of filler particles and a binder polymer. The filler particles include electrode active material particles that are electrochemically oxidized and reduced. The binder polymer includes a copolymer having a first monomer unit with a contact angle to water of 0 to 49° and a second monomer unit with a contact angle to water of 50 to 130°.

### BRIEF SUMMARY

The present invention relates to an aerosol delivery device according to claim 1 (Implementation 1):
An aerosol delivery device comprising a housing defining a reservoir configured to retain aerosol precursor composition, and contained within the housing a heating element controllable to activate and vaporize components of the aerosol precursor composition; and a power source configured to power the heating element to activate and vaporize components of the aerosol precursor composition, the power source comprising a lithium-ion battery (LiB) having an anode formed of graphite, silicon or lithium titanate (LTO), a cathode formed of lithium nickel manganese cobalt oxide or lithium nickel cobalt aluminum oxide, and a polymer separator between the anode and cathode.
**Example Implementation 2:** The aerosol delivery device of the preceding implementation, wherein the anode is formed of graphite and the cathode is formed of lithium nickel manganese cobalt oxide.
**Example Implementation 3:** The aerosol delivery device of any preceding example implementation, wherein the anode is formed of silicon and the cathode is formed of lithium nickel manganese cobalt oxide.
**Example Implementation 4:** The aerosol delivery device of any preceding example implementation, wherein the anode is formed of LTO and the cathode is formed of lithium nickel manganese cobalt oxide.
**Example Implementation 5:** The aerosol delivery device of any preceding example implementation, wherein the anode is formed of graphite and the cathode is formed of lithium nickel cobalt aluminum oxide.
**Example Implementation 6:** The aerosol delivery device of any preceding example implementation, wherein the cathode is a composite cathode or a metal array cathode.
**Example Implementation 7:** The aerosol delivery device of any preceding example implementation, wherein the polymer separator is a solid-state polymer.
**Example Implementation 8:** The aerosol delivery device of any preceding example implementation, wherein the polymer separator is a single layer polymer or a multilayer polymer including polypropylene and polyethylene polymers.
   The present invention further relates to a control body according to claim 9 (Implementation 9) : A control body coupleable with a cartridge that is equipped with a heating element and contains an aerosol precursor composition, the control body being coupleable with the cartridge to form an aerosol delivery device in which the heating element is configured to activate and vaporize components of the aerosol precursor composition, the control body comprising a power source connected to an electrical load that includes the heating element when the control body is coupled with the cartridge, the power source comprising a lithium-ion battery (LiB) having an anode formed of graphite, silicon or lithium titanate (LTO), a cathode formed of lithium nickel manganese cobalt oxide or lithium nickel cobalt aluminum oxide, and a polymer separator between the anode and cathode; and a microprocessor configured to operate in an active mode in which the control body is coupled with the cartridge, the microprocessor in the active mode being configured to direct power from the power source to the heating element to activate and vaporize components of the aerosol precursor composition.
**Example Implementation 10:** The control body of the preceding implementation, wherein the anode is formed of graphite and the cathode is formed of lithium nickel manganese cobalt oxide.
**Example Implementation 11:** The control body of any preceding example implementation, wherein the anode is formed of silicon and the cathode is formed of lithium nickel manganese cobalt oxide.
**Example Implementation 12:** The control body of any preceding example implementation, wherein the anode is formed of LTO and the cathode is formed of lithium nickel manganese cobalt oxide.
**Example Implementation 13:** The control body of any preceding example implementation, wherein the anode is formed of graphite and the cathode is formed of lithium nickel cobalt aluminum oxide.
**Example Implementation 14:** The control body of any preceding example implementation, wherein the cathode is a composite cathode or a metal array cathode.
**Example Implementation 15:** The control body of any preceding example implementation, wherein the polymer separator is a solid-state polymer.
**Example Implementation 16:** The control body of any preceding example implementation, wherein the polymer separator is a single layer polymer or a multilayer polymer including polypropylene and polyethylene polymers.

These and other features, aspects, and advantages of the present disclosure will be apparent from a reading of the following detailed description together with the accompanying drawings, which are briefly described below.

Other example implementations, aspects and advantages will become apparent from the following detailed description taken in conjunction with the accompanying drawings which illustrate, by way of example, the principles of some described example implementations.

### BRIEF DESCRIPTION OF THE DRAWING(S)

Having thus described the disclosure in the foregoing general terms, reference will now be made to the accompanying drawings, which are not necessarily drawn to scale, and wherein:
Figure 1 illustrates a side view of an aerosol delivery device including a cartridge coupled to a control body according to an example implementation of the present disclosure;
Figure 2 is a partially cut-away view of the aerosol delivery device according to various example implementations; and
Figure 3 more particularly illustrates a power source of the control body, according to various example implementations of the present disclosure.

### DETAILED DESCRIPTION

The present disclosure will now be described more fully hereinafter with reference to example implementations thereof. These example implementations are described so that this disclosure will be thorough and complete, and will fully convey the scope of the disclosure to those skilled in the art. Indeed, the disclosure may be embodied in many different forms and should not be construed as limited to the implementations set forth herein; rather, these implementations are provided so that this disclosure will satisfy applicable legal requirements. As used in the specification and the appended claims, the singular forms "a," "an," "the" and the like include plural referents unless the context clearly dictates otherwise.

As described hereinafter, example implementations of the present disclosure relate to aerosol delivery systems. Aerosol delivery systems according to the present disclosure use electrical energy to heat a material (preferably without combusting the material to any significant degree) to form an inhalable substance; and components of such systems have the form of articles most preferably are sufficiently compact to be considered hand-held devices. That is, use of components of preferred aerosol delivery systems does not result in the production of smoke in the sense that aerosol results principally from byproducts of combustion or pyrolysis of tobacco, but rather, use of those preferred systems results in the production of vapors resulting from volatilization or vaporization of certain components incorporated therein. In some example implementations, components of aerosol delivery systems may be characterized as electronic cigarettes, and those electronic cigarettes most preferably incorporate tobacco and/or components derived from tobacco, and hence deliver tobacco derived components in aerosol form.

Aerosol generating pieces of certain preferred aerosol delivery systems may provide many of the sensations (e.g., inhalation and exhalation rituals, types of tastes or flavors, organoleptic effects, physical feel, use rituals, visual cues such as those provided by visible aerosol, and the like) of smoking a cigarette, cigar or pipe that is employed by lighting and burning tobacco (and hence inhaling tobacco smoke), without any substantial degree of combustion of any component thereof. For example, the user of an aerosol generating piece of the present disclosure can hold and use that piece much like a smoker employs a traditional type of smoking article, draw on one end of that piece for inhalation of aerosol produced by that piece, take or draw puffs at selected intervals of time, and the like.

Aerosol delivery systems of the present disclosure also can be characterized as being vapor-producing articles or medicament delivery articles. Thus, such articles or devices can be adapted so as to provide one or more substances (e.g., flavors and/or pharmaceutical active ingredients) in an inhalable form or state. For example, inhalable substances can be substantially in the form of a vapor (i.e., a substance that is in the gas phase at a temperature lower than its critical point). Alternatively, inhalable substances can be in the form of an aerosol (i.e., a suspension of fine solid particles or liquid droplets in a gas). For purposes of simplicity, the term "aerosol" as used herein is meant to include vapors, gases and aerosols of a form or type suitable for human inhalation, whether or not visible, and whether or not of a form that might be considered to be smoke-like.

Aerosol delivery systems of the present disclosure generally include a number of components provided within an outer body or shell, which may be referred to as a housing. The overall design of the outer body or shell can vary, and the format or configuration of the outer body that can define the overall size and shape of the aerosol delivery device can vary. Typically, an elongated body resembling the shape of a cigarette or cigar can be a formed from a single, unitary housing or the elongated housing can be formed of two or more separable bodies. For example, an aerosol delivery device can comprise an elongated shell or body that can be substantially tubular in shape and, as such, resemble the shape of a conventional cigarette or cigar. In one example, all of the components of the aerosol delivery device are contained within one housing. Alternatively, an aerosol delivery device can comprise two or more housings that are joined and are separable. For example, an aerosol delivery device can possess at one end a control body comprising a housing containing one or more reusable components (e.g., an accumulator such as a rechargeable battery and/or supercapacitor, and various electronics for controlling the operation of that article), and at the other end and removably coupleable thereto, an outer body or shell containing a disposable portion (e.g., a disposable flavor-containing cartridge).

Aerosol delivery systems of the present disclosure most preferably comprise some combination of a power source (i.e., an electrical power source), at least one control component (e.g., means for actuating, controlling, regulating and ceasing power for heat generation, such as by controlling electrical current flow the power source to other components of the article - e.g., a microprocessor, individually or as part of a microcontroller), a heater or heat generation member (e.g., an electrical resistance heating element or other component, which alone or in combination with one or more further elements may be commonly referred to as an "atomizer"), an aerosol precursor composition (e.g., commonly a liquid capable of yielding an aerosol upon application of sufficient heat, such as ingredients commonly referred to as "smoke juice," "e-liquid" and "e-juice"), and a mouthend region or tip for allowing draw upon the aerosol delivery device for aerosol inhalation (e.g., a defined airflow path through the article such that aerosol generated can be withdrawn therefrom upon draw).

More specific formats, configurations and arrangements of components within the aerosol delivery systems of the present disclosure will be evident in light of the further disclosure provided hereinafter. Additionally, the selection and arrangement of various aerosol delivery system components can be appreciated upon consideration of the commercially available electronic aerosol delivery devices, such as those representative products referenced in background art section of the present disclosure.

In various examples, an aerosol delivery device can comprise a reservoir configured to retain the aerosol precursor composition. The reservoir particularly can be formed of a porous material (e.g., a fibrous material) and thus may be referred to as a porous substrate (e.g., a fibrous substrate).

A fibrous substrate useful as a reservoir in an aerosol delivery device can be a woven or nonwoven material formed of a plurality of fibers or filaments and can be formed of one or both of natural fibers and synthetic fibers. For example, a fibrous substrate may comprise a fiberglass material. In particular examples, a cellulose acetate material can be used. In other example implementations, a carbon material can be used. A reservoir may be substantially in the form of a container and may include a fibrous material included therein.

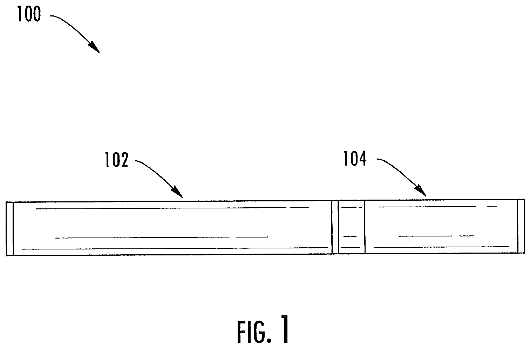

Figure 1 illustrates a side view of an aerosol delivery device **100** including a control body **102** and a cartridge **104,** according to various example implementations of the present disclosure. In particular, Figure 1 illustrates the control body and the cartridge coupled to one another. The control body and the cartridge may be detachably aligned in a functioning relationship. Various mechanisms may connect the cartridge to the control body to result in a threaded engagement, a press-fit engagement, an interference fit, a magnetic engagement or the like. The aerosol delivery device may be substantially rod-like, substantially tubular shaped, or substantially cylindrically shaped in some example implementations when the cartridge and the control body are in an assembled configuration. The aerosol delivery device may also be substantially rectangular or rhomboidal in cross-section, which may lend itself to greater compatibility with a substantially flat or thin-film power source, such as a power source including a flat battery. The cartridge and control body may include separate, respective housings or outer bodies, which may be formed of any of a number of different materials. The housing may be formed of any suitable, structurally-sound material. In some examples, the housing may be formed of a metal or alloy, such as stainless steel, aluminum or the like. Other suitable materials include various plastics (e.g., polycarbonate), metal-plating over plastic, ceramics and the like.

In some example implementations, one or both of the control body **102** or the cartridge **104** of the aerosol delivery device **100** may be referred to as being disposable or as being reusable. For example, the control body may have a replaceable battery or a rechargeable battery and thus may be combined with any type of recharging technology, including connection to a typical wall outlet, connection to a car charger (i.e., a cigarette lighter receptacle), connection to a computer, such as through a universal serial bus (USB) cable or connector, connection to a photovoltaic cell (sometimes referred to as a solar cell) or solar panel of solar cells, or connection to a RF-to-DC converter. Further, in some example implementations, the cartridge may comprise a single-use cartridge, as disclosed in U.S. Pat. No. 8,910,639 to Chang et al.

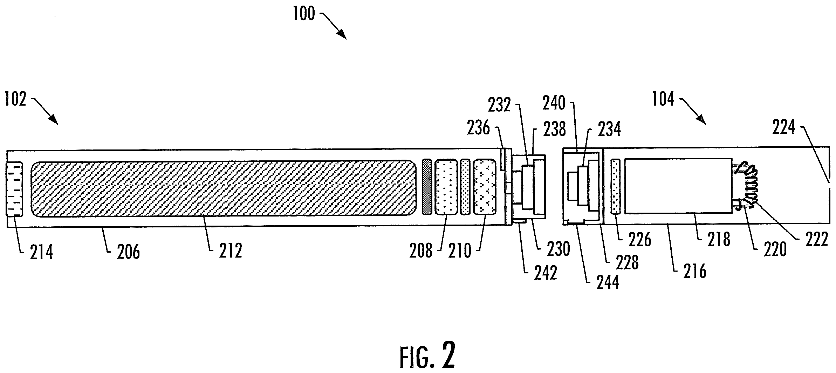

Figure 2 more particularly illustrates the aerosol delivery device **100,** in accordance with some example implementations. As seen in the cut-away view illustrated therein, again, the aerosol delivery device can comprise a control body **102** and a cartridge **104** each of which include a number of respective components. The components illustrated in Figure 2 are representative of the components that may be present in a control body and cartridge and are not intended to limit the scope of components that are encompassed by the present disclosure. As shown, for example, the control body can be formed of a control body shell **206** that can include a control component **208** (e.g., a microprocessor, individually or as part of a microcontroller), a flow sensor **210,** a power source **212** and one or more light-emitting diodes (LEDs) **214,** and such components can be variably aligned. The power source may include, for example, a battery (single-use or rechargeable), lithium-ion battery (LiB), solid-state battery (SSB), thin-film SSB, supercapacitor or the like, or some combination thereof. Some examples of a suitable power source are provided in U.S. Pat. App. Ser. No. 14/918,926 to Sur et al., filed October 21, 2015. The LED may be one example of a suitable visual indicator with which the aerosol delivery device **100** may be equipped. Other indicators such as audio indicators (e.g., speakers), haptic indicators (e.g., vibration motors) or the like can be included in addition to or as an alternative to visual indicators such as the LED.

The cartridge **104** can be formed of a cartridge shell **216** enclosing a reservoir **218** configured to retain the aerosol precursor composition, and including a heater **222** (sometimes referred to as a heating element). In various configurations, this structure may be referred to as a tank; and accordingly, the terms "cartridge," "tank" and the like may be used interchangeably to refer to a shell or other housing enclosing a reservoir for aerosol precursor composition, and including a heater.

As shown, in some examples, the reservoir **218** may be in fluid communication with a liquid transport element **220** adapted to wick or otherwise transport an aerosol precursor composition stored in the reservoir housing to the heater **222.** In some examples, a valve may be positioned between the reservoir and heater, and configured to control an amount of aerosol precursor composition passed or delivered from the reservoir to the heater.

Various examples of materials configured to produce heat when electrical current is applied therethrough may be employed to form the heater **222.** The heater in these examples may be a resistive heating element such as a wire coil, micro heater or the like. Example materials from which the heating element may be formed include Kanthal (FeCrAl), Nichrome, stainless steel, Molybdenum disilicide (MoSi₂), molybdenum silicide (MoSi), Molybdenum disilicide doped with Aluminum (Mo(Si,Al)₂), graphite and graphite-based materials (e.g., carbon-based foams and yarns) and ceramics (e.g., positive or negative temperature coefficient ceramics). Example implementations of heaters or heating members useful in aerosol delivery devices according to the present disclosure are further described below, and can be incorporated into devices such as illustrated in Figure 2 as described herein.

An opening **224** may be present in the cartridge shell **216** (e.g., at the mouthend) to allow for egress of formed aerosol from the cartridge **104.**

The cartridge **104** also may include one or more electronic components **226,** which may include an integrated circuit, a memory component, a sensor, or the like. The electronic components may be adapted to communicate with the control component **208** and/or with an external device by wired or wireless means. The electronic components may be positioned anywhere within the cartridge or a base **228** thereof.

Although the control component **208** and the flow sensor **210** are illustrated separately, it is understood that the control component and the flow sensor may be combined as an electronic circuit board with the air flow sensor attached directly thereto. Further, the electronic circuit board may be positioned horizontally relative the illustration of Figure 1 in that the electronic circuit board can be lengthwise parallel to the central axis of the control body. In some examples, the air flow sensor may comprise its own circuit board or other base element to which it can be attached. In some examples, a flexible circuit board may be utilized. A flexible circuit board may be configured into a variety of shapes, include substantially tubular shapes. In some examples, a flexible circuit board may be combined with, layered onto, or form part or all of a heater substrate as further described below.

The control body **102** and the cartridge **104** may include components adapted to facilitate a fluid engagement therebetween. As illustrated in Figure 2, the control body can include a coupler **230** having a cavity **232** therein. The base **228** of the cartridge can be adapted to engage the coupler and can include a projection **234** adapted to fit within the cavity. Such engagement can facilitate a stable connection between the control body and the cartridge as well as establish an electrical connection between the power source **212** and control component **208** in the control body and the heater **222** in the cartridge. Further, the control body shell **206** can include an air intake **236,** which may be a notch in the shell where it connects to the coupler that allows for passage of ambient air around the coupler and into the shell where it then passes through the cavity **232** of the coupler and into the cartridge through the projection **234.**

A coupler and a base useful according to the present disclosure are described in U.S. Pat. App. Pub. No. 2014/0261495 to Novak et al. For example, the coupler **230** as seen in Figure 2 may define an outer periphery **238** configured to mate with an inner periphery **240** of the base **228.** In one example the inner periphery of the base may define a radius that is substantially equal to, or slightly greater than, a radius of the outer periphery of the coupler. Further, the coupler may define one or more protrusions **242** at the outer periphery configured to engage one or more recesses **244** defined at the inner periphery of the base. However, various other examples of structures, shapes and components may be employed to couple the base to the coupler. In some examples the connection between the base of the cartridge **104** and the coupler of the control body **102** may be substantially permanent, whereas in other examples the connection therebetween may be releasable such that, for example, the control body may be reused with one or more additional cartridges that may be disposable and/or refillable.

The aerosol delivery device **100** may be substantially rod-like or substantially tubular shaped or substantially cylindrically shaped in some examples. In other examples, further shapes and dimensions are encompassed - e.g., a rectangular or triangular cross-section, multifaceted shapes, or the like.

The reservoir **218** illustrated in Figure 2 can be a container or can be a fibrous reservoir, as presently described. For example, the reservoir can comprise one or more layers of nonwoven fibers substantially formed into the shape of a tube encircling the interior of the cartridge shell **216,** in this example. An aerosol precursor composition can be retained in the reservoir. Liquid components, for example, can be sorptively retained by the reservoir. The reservoir can be in fluid connection with the liquid transport element **220.** The liquid transport element can transport the aerosol precursor composition stored in the reservoir via capillary action to the heater **222** that is in the form of a metal wire coil in this example. As such, the heater is in a heating arrangement with the liquid transport element. Example implementations of reservoirs and transport elements useful in aerosol delivery devices according to the present disclosure are further described below, and such reservoirs and/or transport elements can be incorporated into devices such as illustrated in Figure 2 as described herein. In particular, specific combinations of heating members and transport elements as further described below may be incorporated into devices such as illustrated in Figure 2 as described herein.

In use, when a user draws on the aerosol delivery device **100,** airflow is detected by the flow sensor **210,** and the heater **222** is activated to vaporize components of the aerosol precursor composition. Drawing upon the mouthend of the aerosol delivery device causes ambient air to enter the air intake **236** and pass through the cavity **232** in the coupler **230** and the central opening in the projection **234** of the base **228.** In the cartridge **104,** the drawn air combines with the formed vapor to form an aerosol. The aerosol is whisked, aspirated or otherwise drawn away from the heater and out the opening **224** in the mouthend of the aerosol delivery device.

In some examples, the aerosol delivery device **100** may include a number of additional software-controlled functions. For example, the aerosol delivery device may include a power-source protection circuit configured to detect power-source input, loads on the power-source terminals, and charging input. The power-source protection circuit may include short-circuit protection, under-voltage lock out and/or over-voltage charge protection. The aerosol delivery device may also include components for ambient temperature measurement, and its control component **208** may be configured to control at least one functional element to inhibit power-source charging - particularly of any battery - if the ambient temperature is below a certain temperature (e.g., 0 °C) or above a certain temperature (e.g., 45 °C) prior to start of charging or during charging.

Power delivery from the power source **212** may vary over the course of each puff on the device **100** according to a power control mechanism. The device may include a "long puff" safety timer such that in the event that a user or component failure (e.g., flow sensor **210**) causes the device to attempt to puff continuously, the control component **208** may control at least one functional element to terminate the puff automatically after some period of time (e.g., four seconds). Further, the time between puffs on the device may be restricted to less than a period of time (e.g., 100 seconds). A watchdog safety timer may automatically reset the aerosol delivery device if its control component or software running on it becomes unstable and does not service the timer within an appropriate time interval (e.g., eight seconds). Further safety protection may be provided in the event of a defective or otherwise failed flow sensor **210,** such as by permanently disabling the aerosol delivery device in order to prevent inadvertent heating. A puffing limit switch may deactivate the device in the event of a pressure sensor fail causing the device to continuously activate without stopping after the four second maximum puff time.

The aerosol delivery device **100** may include a puff tracking algorithm configured for heater lockout once a defined number of puffs has been achieved for an attached cartridge (based on the number of available puffs calculated in light of the e-liquid charge in the cartridge). The aerosol delivery device may include a sleep, standby or low-power mode function whereby power delivery may be automatically cut off after a defined period of non-use. Further safety protection may be provided in that all charge/discharge cycles of the power source **212** may be monitored by the control component **208** over its lifetime. After the power source has attained the equivalent of a predetermined number (e.g., 200) of full discharge and full recharge cycles, it may be declared depleted, and the control component may control at least one functional element to prevent further charging of the power source.

The various components of an aerosol delivery device according to the present disclosure can be chosen from components described in the art and commercially available. Examples of batteries that can be used according to the disclosure are described in U.S. Pat. App. Pub. No. 2010/0028766 to Peckerar et al.

The aerosol delivery device **100** can incorporate the sensor **210** or another sensor or detector for control of supply of electric power to the heater **222** when aerosol generation is desired (e.g., upon draw during use). As such, for example, there is provided a manner or method of turning off power to the heater when the aerosol delivery device is not be drawn upon during use, and for turning on power to actuate or trigger the generation of heat by the heater during draw. Additional representative types of sensing or detection mechanisms, structure and configuration thereof, components thereof, and general methods of operation thereof, are described in U.S. Pat. No. 5,261,424 to Sprinkel, Jr., U.S. Pat. No. 5,372,148 to McCafferty et al., and PCT Pat. App. Pub. No. WO 2010/003480 to Flick.

The aerosol delivery device **100** most preferably incorporates the control component **208** or another control mechanism for controlling the amount of electric power to the heater **222** during draw. Representative types of electronic components, structure and configuration thereof, features thereof, and general methods of operation thereof, are described in U.S. Pat. No. 4,735,217 to Gerth et al., U.S. Pat. No. 4,947,874 to Brooks et al., U.S. Pat. No. 5,372,148 to McCafferty et al., U.S. Pat. No. 6,040,560 to Fleischhauer et al., U.S. Pat. No. 7,040,314 to Nguyen et al., U.S. Pat. No. 8,205,622 to Pan, U.S. Pat. App. Pub. No. 2009/0230117 to Fernando et al., U.S. Pat. App. Pub. No. 2014/0060554 to Collet et al., U.S. Pat. App. Pub. No. 2014/0270727 to Ampolini et al., and U.S. Pat. App. Ser. No. 14/209,191 to Henry et al.

Representative types of substrates, reservoirs or other components for supporting the aerosol precursor are described in U.S. Pat. No. 8,528,569 to Newton, U.S. Pat. App. Pub. No. 2014/0261487 to Chapman et al., U.S. Pat. App. Ser. No. 14/011,992 to Davis et al., filed August 28, 2013, and U.S. Pat. App. Ser. No. 14/170,838 to Bless et al., filed February 3, 2014. Additionally, various wicking materials, and the configuration and operation of those wicking materials within certain types of electronic cigarettes, are set forth in U.S. Pat. App. Pub. No. 2014/0209105 to Sears et al.

The aerosol precursor composition, also referred to as a vapor precursor composition, may comprise a variety of components including, by way of example, a polyhydric alcohol (e.g., glycerin, propylene glycol or a mixture thereof), nicotine, tobacco, tobacco extract and/or flavorants. Representative types of aerosol precursor components and formulations also are set forth and characterized in U.S. Pat. No. 7,217,320 to Robinson et al. and U.S. Pat. Pub. Nos. 2013/0008457 to Zheng et al.; 2013/0213417 to Chong et al.; 2014/0060554 to Collett et al.; 2015/0020823 to Lipowicz et al.; and 2015/0020830 to Koller, as well as WO 2014/182736 to Bowen et al. Other aerosol precursors that may be employed include the aerosol precursors that have been incorporated in the VUSE® product by R. J. Reynolds Vapor Company, the BLU™ product by Imperial Tobacco Group PLC, the MISTIC MENTHOL product by Mistic Ecigs, and the VYPE product by CN Creative Ltd. Also desirable are the so-called "smoke juices" for electronic cigarettes that have been available from Johnson Creek Enterprises LLC.

Additional representative types of components that yield visual cues or indicators may be employed in the aerosol delivery device **100,** such as visual indicators and related components, audio indicators, haptic indicators and the like. Examples of suitable LED components, and the configurations and uses thereof, are described in U.S. Pat. No. 5,154,192 to Sprinkel et al., U.S. Pat. No. 8,499,766 to Newton, U.S. Pat. No. 8,539,959 to Scatterday, and U.S. Pat. App. Ser. No. 14/173,266 to Sears et al., filed February 5, 2014.

Yet other features, controls or components that can be incorporated into aerosol delivery devices of the present disclosure are described in U.S. Pat. No. 5,967,148 to Harris et al., U.S. Pat. No. 5,934,289 to Watkins et al., U.S. Pat. No. 5,954,979 to Counts et al., U.S. Pat. No. 6,040,560 to Fleischhauer et al., U.S. Pat. No. 8,365,742 to Hon, U.S. Pat. No. 8,402,976 to Fernando et al., U.S. Pat. App. Pub. No. 2005/0016550 to Katase, U.S. Pat. App. Pub. No. 2010/0163063 to Fernando et al., U.S. Pat. App. Pub. No. 2013/0192623 to Tucker et al., U.S. Pat. App. Pub. No. 2013/0298905 to Leven et al., U.S. Pat. App. Pub. No. 2013/0180553 to Kim et al., U.S. Pat. App. Pub. No. 2014/0000638 to Sebastian et al., U.S. Pat. App. Pub. No. 2014/0261495 to Novak et al., and U.S. Pat. App. Pub. No. 2014/0261408 to DePiano et al.

The control component **208** includes a number of electronic components, and in some examples may be formed of a printed circuit board (PCB) that supports and electrically connects the electronic components. The electronic components may include a microprocessor or processor core, and a memory. In some examples, the control component may include a microcontroller with integrated processor core and memory, and may further include one or more integrated input/output peripherals. In some examples, the control component may be coupled to a communication interface **246** to enable wireless communication with one or more networks, computing devices or other appropriately-enabled devices. Examples of suitable communication interfaces are disclosed in U.S. Pat. App. Ser. No. 14/638,562, filed March 4, 2015, to Marion et al., the content of which is incorporated by reference in its entirety. And examples of suitable manners according to which the aerosol delivery device may be configured to wirelessly communicate are disclosed in U.S. Pat. App. Ser. No. 14/327,776, filed July 10, 2014, to Ampolini et al., and U.S. Pat. App. Ser. No. 14/609,032, filed January 29, 2015, to Henry, Jr. et al.

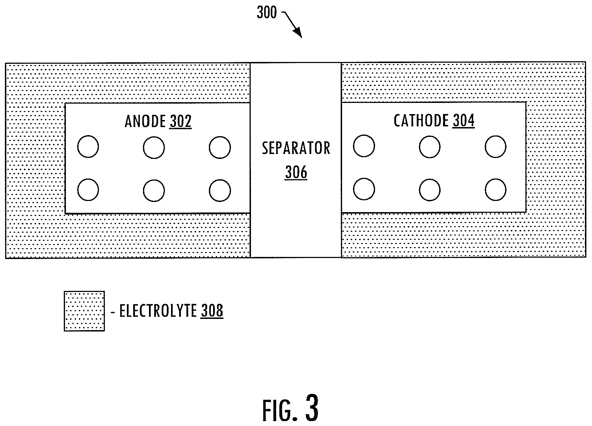

In accordance with some example implementations, the power source **212** may be or include a LiB configured to power the heater **222** to activate and vaporize components of an aerosol precursor composition. Figure 3 more particularly illustrates a LiB **300** that in some examples may correspond to the power source of Figure 2. As shown in Figure 3, the LiB may include an anode **302**, a cathode **304** and a polymer separator **306** therebetween. In particular, the LiB may comprise a number of chemical and/or architectural variations for the anode, cathode and separator in which the anode and cathode may be disposed within an electrolyte **308** that allows ionic conductivity within the LiB.

According to example implementations, the anode **302** may be formed of graphite, silicon or lithium titanate (LTO). The cathode **304** may be formed of lithium nickel manganese cobalt oxide, lithium nickel cobalt aluminum oxide, lithium iron phosphate or lithium manganese oxide. The polymer separator **306** may be a single or multilayer polymer. And the electrolyte **308** may include an organic solvent having lithium salt and additives therein. Any given variation of anodes, cathodes, electrolytes and polymer separators as discussed herein may provide respective benefits for powering the aerosol delivery device **100.** For example, variations on lithium-ion architecture may include varied configurations of anodes, cathodes, electrolytes, and separators, each of which may affect at least the price and performance of the LiB **300**.

In some examples, the anode **302** and cathode **304** may be respectively formed of graphite and lithium nickel manganese cobalt oxide (NMC). In these examples, the NMC cathode may provide improvements to energy density, current density, safety and cost of powering the aerosol delivery device **100.**

In some examples, the anode **302** and cathode **304** may be respectively formed of graphite and lithium nickel cobalt aluminum oxide (NCA). In these examples, the NCA cathode may provide improvements to energy density, current density, safety and cost of powering of an aerosol delivery device **100** having a large-format power source **212.**

In some examples, the anode **302** and cathode **304** may be respectively formed of graphite and lithium iron phosphate (LFP). In these examples, the LFP cathode may provide improvements to safety and sustainability of powering of an aerosol delivery device **100** that requires high load currents and endurance.

In some examples, the anode **302** and cathode **304** may be respectively formed of graphite and lithium manganese oxide (LMO). In these examples, the LMO cathode may provide improvements to safety and sustainability of powering of an aerosol delivery device **100.**

In some of these examples, the anode **302** may be formed of a number of alternative chemical and/or architectural variations as opposed to graphite, silicon or LTO, including aluminum metal, ceramic, hard carbon, lithiated graphite, lithium alloy, lithium aluminum, lithium boron, lithium metal, manganese metal, metallic zinc alloys, metallic zinc, slurried zinc, sodium in graphite carbon, sodium in hard carbon, sodium metal and the like.

In some examples, the cathode **304** may include a composite cathode or an evenly disbursed metal array cathode. Further, in some of these examples, the cathode may be formed of a number of alternative chemical and/or architectural variations as opposed to NMC, NCA, LFP and LMO, including activated carbon, activated carbon and polyvinylidene fluoride, graphite, layered transition metal oxides, lithium cobalt oxide, meso-carbon, molybdenum sulfide, phosphates, porous carbon, porous carbon with a catalyst layer, porous titanium dioxide, sulfur, sulfur on carbon and the like.

In some examples, the LiB **300** may include a single layer, multilayer or solid-state polymer separator **306.** In these examples, the multilayer polymer separator may include a polypropylene and polyethylene polymer, and the multilayer polymer separator may provide significant improvements to safety without imposing a negative impact on performance of the LiB.

In some examples, the polymer separator **306** may be formed of a number of alternative chemical and/or architectural variations as opposed to a single, multilayer, or solid-state polymer such as polytetrafluoroethylene or polyvinyl alcohol. Further in some of these examples, the separator may include a non-polymer separator such as ceramic (e.g., aluminum ion conducting ceramics), ceramic foam, glass fiber, glass microfiber, graphene oxide, non-woven cellulose material, paper, and the like.

In some examples, the electrolyte **308** may be formed of a number of alternative chemical and/or architectural variations as opposed to an organic solvent having lithium salt and additives therein, including a range of organic, aqueous, mixed organic and/or aqueous, solid-state electrolytes; a porous polymer gel, aluminum chloride in ionic liquid, diethylene glycol dimethyl ether, fluorinated carbonates or ionic liquids, lithium phosphate, lithium phosphorus oxynitride, lithium salt mixed with polyethylene oxide or polyvinylidene fluoride, magnesium organo-haloaluminte salts, polysulfide, potassium hydroxide, sodium hexafluorophosphate in a mixture of propylene carbonate and ethylene carbonate, sodium hydroxide, sodium perchlorate, sodium perchlorate-propylene carbonate, tetraethylene glycol dimethyl ether, and the like.

The foregoing description of use of the article(s) can be applied to the various example implementations described herein through minor modifications, which can be apparent to the person of skill in the art in light of the further disclosure provided herein. The above description of use, however, is not intended to limit the use of the article but is provided to comply with all necessary requirements of disclosure of the present disclosure. Any of the elements shown in the article(s) illustrated in Figures 1-3 or as otherwise described above may be included in an aerosol delivery device according to the present disclosure.

Many modifications and other implementations of the disclosure set forth herein will come to mind to one skilled in the art to which this disclosure pertains having the benefit of the teachings presented in the foregoing descriptions and the associated drawings. Therefore, it is to be understood that the disclosure is not to be limited to the specific implementations disclosed, and that modifications and other implementations are intended to be included within the scope of the appended claims. Moreover, although the foregoing descriptions and the associated drawings describe example implementations in the context of certain example combinations of elements and/or functions, it should be appreciated that different combinations of elements and/or functions may be provided by alternative implementations without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An aerosol delivery device (100) comprising a housing defining a reservoir configured to retain aerosol precursor composition, and contained within the housing:
a heating element (222) controllable to activate and vaporize components of the aerosol precursor composition; and
a power source (212) configured to power the heating element (222) to activate and vaporize components of the aerosol precursor composition, the power source (212) comprising a lithium-ion battery (LiB) (300) having an anode (302) formed of graphite, silicon or lithium titanate (LTO), a cathode (304) formed of lithium nickel manganese cobalt oxide or lithium nickel cobalt aluminum oxide, and a polymer separator (306) between the anode (302) and cathode (304).

2. The aerosol delivery device (100) of Claim 1, wherein the anode (302) is formed of graphite and the cathode (304) is formed of lithium nickel manganese cobalt oxide.

3. The aerosol delivery device (100) of any one of Claims 1 or 2, wherein the anode (302) is formed of silicon and the cathode (304) is formed of lithium nickel manganese cobalt oxide.

4. The aerosol delivery device (100) of any one of Claims 1 to 3, wherein the anode (302) is formed of LTO and the cathode (304) is formed of lithium nickel manganese cobalt oxide.

5. The aerosol delivery device (100) of any one of Claims 1 to 4, wherein the anode (302) is formed of graphite and the cathode (304) is formed of lithium nickel cobalt aluminum oxide.

6. The aerosol delivery device (100) of any one of Claims 1 to 5, wherein the cathode (304) is a composite cathode or a metal array cathode.

7. The aerosol delivery device (100) of any one of Claims 1 to 6, wherein the polymer separator (306) is a solid-state polymer.

8. The aerosol delivery device (100) of any one of Claims 1 to 7, wherein the polymer separator (306) is a single layer polymer or a multilayer polymer including polypropylene and polyethylene polymers.

9. A control body (102) coupleable with a cartridge (104) that is equipped with a heating element (222) and contains an aerosol precursor composition, the control body (102) being coupleable with the cartridge (104) to form an aerosol delivery device (100) in which the heating element (222) is configured to activate and vaporize components of the aerosol precursor composition, the control body (102) comprising:
a power source (212) connected to an electrical load that includes the heating element (222) when the control body (102) is coupled with the cartridge (104), the power source (212) comprising a lithium-ion battery (LiB) (300) having an anode (302) formed of graphite, silicon or lithium titanate (LTO), a cathode (304) formed of lithium nickel manganese cobalt oxide or lithium nickel cobalt aluminum oxide, and a polymer separator (306) between the anode (302) and cathode (304); and
a microprocessor (208) configured to operate in an active mode in which the control body (102) is coupled with the cartridge (104), the microprocessor (208) in the active mode being configured to direct power from the power source (212) to the heating element (222) to activate and vaporize components of the aerosol precursor composition.

10. The control body (102) of Claim 9, wherein the anode (302) is formed of graphite and the cathode (304) is formed of lithium nickel manganese cobalt oxide.

11. The control body (102) of Claim 9 or 10, wherein the anode (302) is formed of silicon and the cathode (304) is formed of lithium nickel manganese cobalt oxide.

12. The control body (102) of any one of Claims 9 to 11, wherein the anode (302) is formed of LTO and the cathode (304) is formed of lithium nickel manganese cobalt oxide.

13. The control body (102) of any one of Claims 9 to 12, wherein the anode (302) is formed of graphite and the cathode (304) is formed of lithium nickel cobalt aluminum oxide.

14. The control body (102) of any one of Claims 9 to 13, wherein the cathode (304) is a composite cathode or a metal array cathode.

15. The control body (102) of any one of Claims 9 to 14, wherein the polymer separator (306) is a solid-state polymer.

16. The control body (102) of any one of Claims 9 to 15, wherein the polymer separator (306) is a single layer polymer or a multilayer polymer including polypropylene and polyethylene polymers.

## Patentansprüche

1. Eine Aerosolabgabevorrichtung (100), umfassend ein Gehäuse, welches ein Reservoir definiert, das ausgebildet ist, eine Aerosol-Precursor-Zusammensetzung zu halten, und - aufgenommen innerhalb des Gehäuses -:
ein Heizelement (222), welches kontrollierbar ist, um Komponenten der Aerosol-Precursor-Zusammensetzung zu aktivieren und zu verdampfen; und
eine Leistungsquelle (212), welche dazu ausgebildet ist, das Heizelement (222) mit Leistung zu versorgen, um Komponenten der Aerosol-Precursor-Zusammensetzung zu aktivieren und zu verdampfen, wobei die Leistungsquelle (212) eine Lithium-Ionen-Batterie (LiB) (300) umfasst, aufweisend eine Anode (302), welche von Graphit, Silicium oder Lithiumtitanat (LTO) gebildet ist, eine Kathode (304), welche von Lithium-Nickel-Mangan-Cobalt-Oxid oder Lithium-Nickel-Cobalt-Aluminium-Oxid gebildet ist, und einen Polymerseparator (306) zwischen der Anode (302) und der Kathode (304).

2. Die Aerosolabgabevorrichtung (100) nach Anspruch 1, wobei die Anode (302) von Graphit gebildet ist und die Kathode (304) von Lithium-Nickel-Mangan-Cobalt-Oxid gebildet ist.

3. Die Aerosolabgabevorrichtung (100) nach einem der Ansprüche 1 oder 2, wobei die Anode (302) von Silicium gebildet ist und die Kathode (304) von Lithium-Nickel-Mangan-Cobalt-Oxid gebildet ist.

4. Die Aerosolabgabevorrichtung (100) nach einem der Ansprüche 1 bis 3, wobei die Anode (302) von LTO gebildet ist und die Kathode (304) von Lithium-Nickel-Mangan-Cobalt-Oxid gebildet ist.

5. Die Aerosolabgabevorrichtung (100) nach einem der Ansprüche 1 bis 4, wobei die Anode (302) von Graphit gebildet ist und die Kathode (304) von Lithium-Nickel-Cobalt-Aluminium-Oxid gebildet ist.

6. Die Aerosolabgabevorrichtung (100) nach einem der Ansprüche 1 bis 5, wobei die Kathode (304) eine Komposit-Kathode oder eine Metall-Array-Kathode ist.

7. Die Aerosolabgabevorrichtung (100) nach einem der Ansprüche 1 bis 6, wobei der Polymerseparator (306) ein Festkörperpolymer ist.

8. Die Aerosolabgabevorrichtung (100) nach einem der Ansprüche 1 bis 7, wobei der Polymerseparator (306) ein Einlagen-Polymer oder ein Mehrlagen-Polymer ist, welches Polypropylen- und Polyethylen-Polymere umfasst.

9. Ein Kontrollkörper (102), welcher mit einer Patrone (104) koppelbar ist, welche mit einem Heizelement (222) ausgestattet ist und eine Aerosol-Precursor-Zusammensetzung enthält, wobei der Kontrollkörper (102) mit der Patrone (104) koppelbar ist, um eine Aerosolabgabevorrichtung (100) zu bilden, bei welcher das Heizelement (222) ausgebildet ist, Komponenten der Aerosol-Precursor-Zusammensetzung zu aktivieren und zu verdampfen, wobei der Kontrollkörper (102) umfasst:
eine Leistungsquelle (212), die mit einer elektrischen Last verbunden ist, welche das Heizelement (222) umfasst, wenn der Kontrollkörper (102) mit der Patrone (104) gekoppelt ist, wobei die Leistungsquelle (212) eine Lithium-Ionen-Batterie (LiB) (300) umfasst, aufweisend eine Anode (302), welche von Graphit, Silicium oder Lithiumtitanat (LTO) gebildet ist, eine Kathode (304), welche von Lithium-Nickel-Mangan-Cobalt-Oxid oder Lithium-Nickel-Cobalt-Aluminium-Oxid gebildet ist, und einen Polymerseparator (306) zwischen der Anode (302) und der Kathode (304); und
einen Mikroprozessor (208), welcher dazu ausgebildet ist, in einem aktiven Modus zu arbeiten, in dem der Kontrollkörper (102) mit der Patrone (104) gekoppelt ist, wobei der Mikroprozessor (208) in dem aktiven Modus dazu ausgebildet ist, Leistung von der Leistungsquelle (212) zu dem Heizelement (222) zu richten, um Komponenten der Aerosol-Precursor-Zusammensetzung zu aktivieren und zu verdampfen.

10. Der Kontrollkörper (102) nach Anspruch 9, wobei die Anode (302) von Graphit gebildet ist und die Kathode (304) von Lithium-Nickel-Mangan-Cobalt-Oxid gebildet ist.

11. Der Kontrollkörper (102) nach Anspruch 9 oder 10, wobei die Anode (302) von Silicium gebildet ist und die Kathode (304) von Lithium-Nickel-Mangan-Cobalt-Oxid gebildet ist.

12. Der Kontrollkörper (102) nach einem der Ansprüche 9 bis 11, wobei die Anode (302) von LTO gebildet ist und die Kathode (304) von Lithium-Nickel-Mangan-Cobalt-Oxid gebildet ist.

13. Der Kontrollkörper (102) nach einem der Ansprüche 9 bis 12, wobei die Anode (302) von Graphit gebildet ist und die Kathode (304) von Lithium-Nickel-Cobalt-Aluminium-Oxid gebildet ist.

14. Der Kontrollkörper (102) nach einem der Ansprüche 9 bis 13, wobei die Kathode (304) eine Komposit-Kathode oder eine Metall-Array-Kathode ist.

15. Der Kontrollkörper (102) nach einem der Ansprüche 9 bis 14, wobei der Polymerseparator (306) ein Festkörperpolymer ist.

16. Der Kontrollkörper (102) nach einem der Ansprüche 9 bis 15, wobei der Polymerseparator (306) ein Einlagen-Polymer oder ein Mehrlagen-Polymer ist, welches Polypropylen- und Polyethylen-Polymere umfasst.

## Revendications

1. Dispositif de délivrance d'aérosol (100) comprenant un boîtier définissant un réservoir configuré pour retenir une composition de précurseur d'aérosol, et contenu dans le boîtier ;
un élément chauffant (222) pouvant être commandé afin qu'il active et vaporise des composants de la composition de précurseur d'aérosol ; et
une source d'alimentation (212) configurée pour alimenter l'élément chauffant (222) afin qu'il active et vaporise des composants de la composition de précurseur d'aérosol, la source d'alimentation (212) comprenant une batterie au lithium-ion (LiB) (300) ayant une anode (302) formée de graphite, de silicium ou de titanate de lithium (LTO), une cathode (304) formée d'oxyde de lithium, nickel, manganèse et cobalt, ou d'oxyde de lithium, nickel, cobalt et aluminium, et un séparateur polymère (306) entre l'anode (302) et la cathode (304).

2. Dispositif de délivrance d'aérosol (100) selon la revendication 1, dans lequel l'anode (302) est formée de graphite et la cathode (304) est formée d'oxyde de lithium, nickel, manganèse et cobalt.

3. Dispositif de délivrance d'aérosol (100) selon l'une quelconque des revendications 1 ou 2, dans lequel l'anode (302) est formée de silicium et la cathode (304) est formée d'oxyde de lithium, nickel, manganèse et cobalt.

4. Dispositif de délivrance d'aérosol (100) selon l'une quelconque des revendications 1 à 3, dans lequel l'anode (302) est formée de LTO et la cathode (304) est formée d'oxyde de lithium, nickel, manganèse et cobalt.

5. Dispositif de délivrance d'aérosol (100) selon l'une quelconque des revendications 1 à 4, dans lequel l'anode (302) est formée de graphite et la cathode (304) est formée d'oxyde de lithium, nickel, cobalt et aluminium.

6. Dispositif de délivrance d'aérosol (100) selon l'une quelconque des revendications 1 à 5, dans lequel la cathode (304) est une cathode composite ou une cathode en réseau métallique.

7. Dispositif de délivrance d'aérosol (100) selon l'une quelconque des revendications 1 à 6, dans lequel le séparateur polymère (306) est un polymère à l'état solide.

8. Dispositif de délivrance d'aérosol (100) selon l'une quelconque des revendications 1 à 7, dans lequel le séparateur polymère (306) est un polymère monocouche ou un polymère multicouche comprenant des polymères de polypropylène et de polyéthylène.

9. Corps de commande (102) pouvant être couplé avec une cartouche (104) qui est équipée d'un élément chauffant (222) et contient une composition de précurseur d'aérosol, le corps de commande (102) pouvant être couplé avec la cartouche (104) pour former un dispositif de délivrance d'aérosol (100) dans lequel l'élément chauffant (222) est configuré pour activer et vaporiser des composants de la composition de précurseur d'aérosol, le corps de commande (102) comprenant :
une source d'alimentation (212) connectée à une charge électrique qui comprend l'élément chauffant (222) quand le corps de commande (102) est couplé avec la cartouche (104), la source d'alimentation (212) comprenant une batterie au lithium-ion (LiB) (300) ayant une anode (302) formée de graphite, de silicium ou de titanate de lithium (LTO), une cathode (304) formée d'oxyde de lithium, nickel, manganèse et cobalt, ou d'oxyde de lithium, nickel, cobalt et aluminium, et un séparateur polymère (306) entre l'anode (302) et la cathode (304); et
un microprocesseur (208) configuré pour fonctionner selon un mode actif dans lequel le corps de commande (102) est couplé avec la cartouche (104), le microprocesseur (208) en mode actif étant configuré pour diriger l'énergie depuis la source d'alimentation (212) vers l'élément chauffant (222) pour qu'il active et vaporise des composants de la composition de précurseur d'aérosol.

10. Corps de commande (102) selon la revendication 9, dans lequel l'anode (302) est formée de graphite et la cathode (304) est formée d'oxyde de lithium, nickel, manganèse et cobalt.

11. Corps de commande (102) selon la revendication 9 ou 10, dans lequel l'anode (302) est formée de silicium et la cathode (304) est formée d'oxyde de lithium, nickel, manganèse et cobalt.

12. Corps de commande (102) selon l'une quelconque des revendications 9 à 11, dans lequel l'anode (302) est formée de LTO et la cathode (304) est formée d'oxyde de lithium, nickel, manganèse et cobalt.

13. Corps de commande (102) selon l'une quelconque des revendications 9 à 12, dans lequel l'anode (302) est formée de graphite et la cathode (304) est formée d'oxyde de lithium, nickel, cobalt et aluminium.

14. Corps de commande (102) selon l'une quelconque des revendications 9 à 13, dans lequel la cathode (304) est une cathode composite ou une cathode en réseau métallique.

15. Corps de commande (102) selon l'une quelconque des revendications 9 à 14, dans lequel le séparateur polymère (306) est un polymère à l'état solide.

16. Corps de commande (102) selon l'une quelconque des revendications 9 à 15, dans lequel le séparateur polymère (306) est un polymère monocouche ou un polymère multicouche comprenant des polymères de polypropylène et de polyéthylène.
